# FASCICULE DE BREVET EUROPEEN

(11) **EP 3 046 501 B1**
(45) Date de publication et mention de la délivrance du brevet: **17.07.2019**
(21) Numéro de dépôt: 14789888.6
(22) Date de dépôt: 19.09.2014
(51) Int. Cl.: A61C 7/00

(54) **PROCÉDÉ DE CONTRÔLE DU POSITIONNEMENT DE DENTS**
VERFAHREN ZUR ÜBERWACHUNG DER POSITION VON ZÄHNEN
METHOD FOR MONITORING THE POSITION OF TEETH

(30) Priorité: 19.09.2013 FR 1359038
(43) Date de publication de la demande: 27.07.2016
(73) Titulaire: Dental Monitoring S.A.S., 75008 Paris (FR)
(72) Inventeur: SALAH, Emmanuel, FRANCE Aulnay sous Bois (FR); AYACHE, William, 92200 Neuilly sur Seine (FR); SALAH, Philippe, 75020 Paris (FR)
(74) Mandataire: Nony
(86) Numéro de dépôt international: PCT/IB2014/064658
(87) Numéro de publication internationale: WO 2015/040577

(56) Documents cités:
- CA-A1- 2 292 141
- JP-B- 5 241 971
- US-A1- 2006 136 267
- US-A1- 2008 306 724
- US-A1- 2010 042 440
- TANER T ( ET AL: "Evaluation of dental arch width and form changes after orthodontic treatment and retention with a new computerized method", AMERICAN JOURNAL OF ORTHODONTICS AND DENTOFACIAL ORTHOPE, MOSBY, ST. LOUIS, MO, US, vol. 126, no. 4, 1 October 2004 (2004-10-01), pages 463-474, XP004584030, ISSN: 0889-5406, DOI: 10.1016/J.AJODO.2003.08.033

## Description

### Domaine technique

La présente invention concerne un procédé de contrôle du positionnement de dents d'un patient et un programme informatique pour la mise en oeuvre de ce procédé.

### Etat de la technique

Après un traitement orthodontique, il est nécessaire que le patient traité fasse régulièrement contrôler sa dentition, notamment afin de vérifier que la position des dents n'évolue pas défavorablement. Cette évolution défavorable est encore appelée « récidive ». Classiquement, le patient se rend donc à intervalles réguliers chez son orthodontiste afin d'effectuer ces contrôles. Il peut également se rendre chez son dentiste, à même de détecter toute imperfection dans le positionnement des dents.

De nombreux patients n'effectuent pas ces visites de contrôle, qui permettraient la détection d'éventuelles situations de récidive. Les dents peuvent ainsi reprendre une position de mal occlusion qui, pour être corrigée, nécessite un nouveau traitement orthodontique, pouvant être aussi important que le traitement initial.

En outre, les visites sont contraignantes pour le patient et constituent un élément de stress pour l'orthodontiste.

L'article "Evaluation of dental arch width and form changes after orthodontic treatment and retention with a new computerized method" par Tülin Taner publié dans l'American journal of Orthodontie and Dentofacial Orthopedics (Volume 126, Nr. 4 / XP004584030), décrit une méthode d'étude orthodontique incluant un procédé de contrôle du positionnement de dents d'un patient afin de contrôler la récidive après un traitement orthodontique, ledit procédé comportant les étapes suivantes: a) moulage des dents après traitement orthodontique à T1 et scan dudit moulage; b) après un intervalle de temps T2= T1+1an moulage des dents et scan dudit moulage; c) comparaison, par un programme d'ordinateur, desdits modèles cible (T1) et actualisé (T2).

Un objectif de la présente invention est de répondre, au moins partiellement, aux problèmes susmentionnés.

### Résumé de l'invention

L'invention fournit un procédé de contrôle du positionnement de dents d'un patient afin de contrôler la récidive après un traitement orthodontique, ledit procédé comportant les étapes suivantes :
a) moins de 6 mois après la fin dudit traitement orthodontique, modélisation d'un positionnement cible desdites dents sous la forme d'un modèle cible, au moyen d'un appareil professionnel, le modèle cible étant un modèle des dents dans leur position corrigée, résultant de la mise en oeuvre du traitement orthodontique;
b) après un intervalle de temps, modélisation d'un positionnement actualisé desdites dents sous la forme d'un modèle actualisé au moyen d'un appareil personnel;
c) comparaison, par un programme d'ordinateur, desdits modèles cible et actualisé.

Un procédé selon l'invention peut encore comporter une ou plusieurs des caractéristiques optionnelles suivantes :
- le modèle cible est un modèle tridimensionnel ;
- le modèle cible fournit une information sur le positionnement des dents avec une erreur inférieure à 5/10 mm, de préférence inférieure à 3/10 mm, de préférence inférieure à 1/10 mm ;
- l'intervalle de temps est supérieur à 1 mois et/ou est déterminé par le patient ou, de préférence, par un orthodontiste;
- l'étape b) est réalisée à distance de l'étape a), c'est-à-dire dans un lieu différent de celui dans lequel est réalisée l'étape a), en particulier à plus de 50 m, plus de 100 m, plus de 1 km du lieu où est réalisée l'étape a), en particulier hors cabinet orthodontique ;
- l'étape b) n'est pas réalisée dans un cabinet dentaire, un cabinet d'orthodontie ou un laboratoire d'orthodontie ;
- l'appareil personnel est un appareil individuel choisi dans le groupe formé par un téléphone mobile, un appareil photo dit « connecté », une montre dite « intelligente », ou « smartwatch », une tablette numérique, un scanner 3D portable et un ordinateur couplé un système d'acquisition d'images, telle une webcam ou un appareil photo numérique, pour mettre en oeuvre l'étape b) et/ou l'étape c), de préférence l'étape b) et l'étape c) ;
- le modèle actualisé comporte une vidéo ou une photo des dents du patient, de préférence une photo prise face au patient, une photo prise du côté droit du patient et une photo prise du côté gauche du patient ;
- le modèle actualisé comporte une vidéo des dents du patient ;
- l'étape b) est de préférence réalisée par le patient ou un proche du patient, mais peut être réalisée par un dentiste ;
- à l'étape b), une ou plusieurs photos et/ou vidéos des dents du patient sont traitées afin de créer un modèle actualisé tridimensionnel ;
- préalablement à l'étape c), de préférence préalablement à l'étape b), le patient ou un professionnel des soins dentaires charge le modèle cible dans ledit appareil individuel ou le met à disposition pour chargeaient dans ledit appareil individuel ; le patient charge de préférence le modèle cible à partir d'Internet ;
- à l'étape c), on génère une information dont le contenu dépend de la différence ou "distance" entre les modèles ciblé et actualisé et, de préférence, on transmet cette information audit patient et/ou à un professionnel des soins dentaires ;
- en fonction de la comparaison à l'étape c), on informe le patient et/ou un professionnel des soins dentaires, de la probable nécessité d'une consultation d'un dentiste ou d'un orthodontiste ;
- l'étape c) de comparaison des modèles est réalisée soit sur un appareil personnel du patient, soit avec un applicatif chez un professionnel des soins dentaires, soit avec un serveur tierce dédié.

L'invention concerne aussi l'utilisation d'un procédé selon l'invention pour
- déterminer une vitesse d'évolution d'un changement de positions des dents, et/ou
- optimiser la date de prise de rendez-vous chez un orthodontiste ou un dentiste, et/ou
- évaluer l'efficacité d'un traitement orthodontique.

L'invention concerne également un programme d'ordinateur, et en particulier un applicatif spécialisé configuré pour la mise en oeuvre de l'étape b) et/ou de l'étape c), de préférence des deux étapes b) et c), un support informatique sur lequel est enregistré un tel programme informatique, par exemple une mémoire ou un CD-ROM, et un appareil personnel, en particulier un téléphone mobile ou une tablette, dans lequel est chargé un tel programme.

L'appareil personnel peut en particulier comporter une module d'acquisition et/ou un module de traitement et/ou un module de comparaison et/ou un module de communication comportant une ou plusieurs des caractéristiques décrites ci-après, de manière à pouvoir exécuter une ou plusieurs des opérations d'un procédé selon l'invention.

L'invention peut être mise en oeuvre dans un système comportant un appareil équipant un professionnel, apte à mettre en oeuvre l'étape a) d'un procédé selon l'invention, de préférence pour créer un modèle tridimensionnel de la dentition dudit patient, un appareil personnel équipant un patient, de préférence d'un téléphone mobile, chargé avec un applicatif spécialisé apte à mettre en oeuvre des étapes b) et c) d'un procédé selon invention.

### Définitions

Par « patient », on entend toute personne pour laquelle un procédé est mis en oeuvre afin d'en contrôler les dents, que cette personne soit malade ou non.
Par « professionnel des soins dentaires », on entend un dentiste, un orthodontiste ou un laboratoire d'orthodontie.
Par « dentiste », on entend un dentiste ou un assistant dentaire travaillant sous la responsabilité d'un dentiste.
On appelle "téléphone mobile" un appareil de moins de 500 g, doté d'un capteur lui permettant de capturer des images, capable d'échanger des données avec un autre appareil éloigné de plus de 500 km du téléphone mobile, et capable d'afficher lesdites données, et notamment lesdites images. Une « tablette » est un ordinateur portable à écran tactile.

Un scanner 3D est un appareil permettant d'obtenir une représentation en trois dimensions d'un objet.

Par "comprenant un" ou "comportant un", on entend "comportant au moins un", sauf indication contraire.

### Brève description des figures

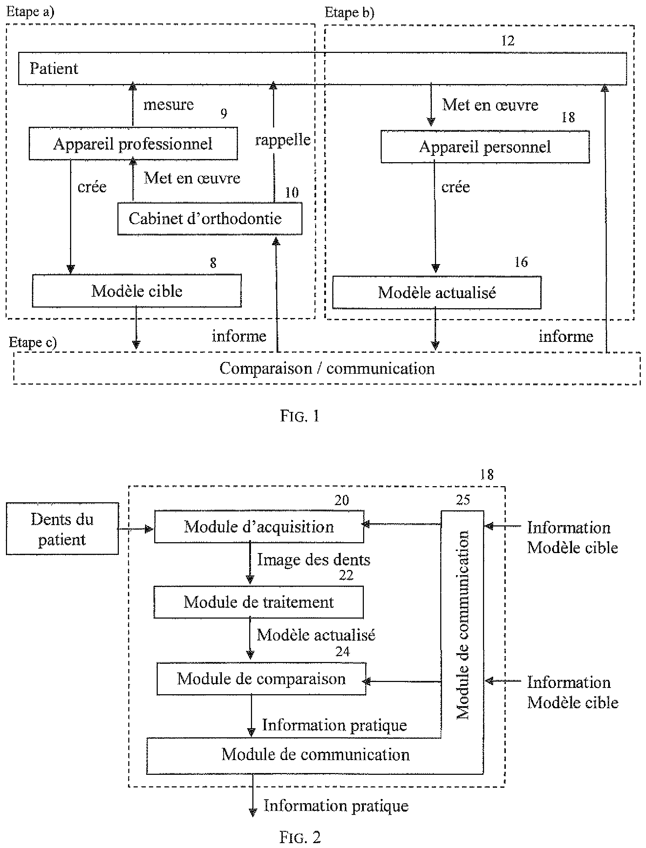

D'autres caractéristiques et avantages de l'invention apparaîtront encore à la lecture de la description détaillée qui va suivre et à l'examen du dessin annexé dans lequel la figure 1 et la figure 2 illustrent respectivement un procédé et le contenu d'un téléphone mobile pouvant être utilisé pour la mise en oeuvre d'un procédé selon l'invention.

### Description détaillée

Le procédé de la présente invention est défini par les revendications 1-17 et comporte trois étapes a), b) et c) mentionnées dans la revendication 1.

**A l'étape a**), un modèle cible 8 des dents du patient est créé (voir figure 1).

Dans le cadre d'un contrôle de la récidive, le modèle cible est un modèle des dents dans leur position corrigée, résultant de la mise en oeuvre du traitement orthodontique. Le modèle cible est donc effectué après le traitement orthodontique, c'est-à-dire à un moment où le patient ne porte plus d'appareil de contention orthodontique actif.

Le modèle cible peut être alors également appelé modèle "initial".

De préférence, le modèle cible est préparé moins de six mois, de préférence moins de trois mois, de préférence encore moins de un mois après la fin du traitement orthodontique, généralement immédiatement après la fin du traitement. Il correspond ainsi à un positionnement sensiblement optimal des dents.

La forme du modèle cible n'est pas limitée. Il peut s'agir en particulier d'un modèle numérique en trois dimensions, mais aussi d'une image, d'une photo en deux dimensions, par exemple une photo panoramique, un film. Dans un mode de réalisation, le modèle cible peut être constitué par une ou plusieurs mesures effectuées sur les dents, par exemple la mesure d'un écartement entre deux dents adjacentes.

De préférence, le modèle cible est un modèle numérique à trois dimensions de la dentition du patient, par exemple du type .stl ou .Obj, .DXF 3D, IGES, STEP, VDA, ou Nuages de points. Avantageusement, un tel modèle, dit « 3D », peut être observé selon un angle quelconque.

Le modèle cible peut être préparé à partir de mesures effectuées sur les dents du patient ou sur un modèle physique de ses dents, par exemple un modèle en plâtre.

Le modèle cible est créé au moyen d'un appareil professionnel 9, de préférence dans un Cabinet d'orthodontie, par exemple au moyen d'un scanner 3D, de préférence mis en oeuvre par un professionnel de la santé, par exemple par un orthodontiste ou un laboratoire d'orthodontie 10. Dans un cabinet d'orthodontie, le patient 12 ou le modèle physique de ses dents peuvent être avantageusement disposés dans une position précise et les moyens d'acquisition peuvent être perfectionnés. Il en résulte un modèle cible très précis.

Le modèle cible peut être stocké dans une base de données centralisée, regroupant les modèles cible d'une pluralité de patients. Cette base de données peut être physiquement installée dans un établissement spécialisé. Elle peut être également installée dans un laboratoire ou un cabinet d'orthodontie, ce qui limite les transferts d'informations confidentielles.

Dans un mode de réalisation, le modèle cible, ou une copie du modèle cible, est remis au patient. De préférence, un fichier informatique correspondant au modèle cible est enregistré sur un support amovible, par exemple sur une clé USB ou sur une carte électronique, de préférence sur un téléphone mobile, une tablette ou un ordinateur portable du patient, et en particulier sur l'appareil personnel qui sera utilisé à l'étape b).

**A l'étape b),** après un intervalle de temps Δt, un modèle actualisé 16 est créé.

L'intervalle de temps Δt peut être prédéterminé. Il peut être constant, quelle que soit l'occurrence du procédé, c'est-à-dire que cet intervalle concerne la première exécution du procédé ou une exécution ultérieure. Il peut être variable, et dépendre par exemple des résultats d'une étape c) antérieure. En particulier, l'intervalle de temps Δt peut être d'autant plus court que cette étape c) a détecté une dérive importante.

Dans un mode de réalisation préféré, l'intervalle de temps Δt est déterminé par l'orthodontiste, en fonction d'un planning des contrôles. En fonction de l'évolution de la position des dents, l'orthodontiste peut modifier ce planning et modifier en conséquence l'intervalle de temps Δt. Dans un mode de réalisation, le procédé de contrôle met en oeuvre plusieurs cycles comportant chacun une étape b) et une étape c), les intervalles de temps entre chaque cycle pouvant être identiques ou différents. Les intervalles des temps entre deux cycles successifs peuvent être tous déterminés avant l'exécution du premier cycle pour correspondre à un planning de contrôles élaboré par l'orthodontiste.

L'intervalle de temps Δt peut être également indéterminé et dépendre par exemple de décisions du patient. Par exemple, la création d'un modèle actualisé peut être effectuée à l'occasion d'un rendez-vous chez le dentiste ou à tout moment lorsque le patient le souhaite.

L'intervalle de temps Δt est de préférence déterminé pour correspondre à une évolution potentiellement significative du positionnement des dents. De préférence, la première année après le traitement, l'intervalle de temps Δt est inférieur à trois mois. Après cette première année, l'intervalle de temps Δt est de préférence supérieur à un mois, voire supérieur à six mois ou supérieur à douze mois. En particulier pour la détection d'une dérive des dents, un intervalle de temps compris entre six mois et dix-huit mois est adapté. De préférence, au moins un rappel informant le patient de la nécessité de créer un modèle actualisé est adressé au patient. Ce rappel peut être sous forme papier ou, de préférence, sous forme électronique, par exemple sous la forme d'un courriel, d'une alerte automatique de l'applicatif spécialisé mobile ou d'un SMS. Un tel rappel peut être envoyé par le cabinet ou le laboratoire d'orthodontie 10, ou par le dentiste, par exemple.

Le modèle actualisé peut être préparé comme le modèle cible ou non. Il peut avoir un format identique ou différent de celui du modèle cible.

Le modèle actualisé 16 est créé avec un appareil personnel 18 couramment disponible dans le commerce, par exemple un téléphone mobile ou une tablette ou un ordinateur personnel, fixe ou portable, de préférence un appareil photo. Le modèle actualisé 16 peut en particulier être créé par le patient lui-même ou par un de ses proches. A cet effet, un applicatif spécialisé est de préférence chargé dans l'appareil personnel 18.

L'appareil personnel pèse de préférence moins de 3 kg, moins de 2 kg, moins de 1 kg, moins de 500 g, de préférence moins de 300 g.

De préférence encore, le modèle actualisé 16 est constitué à partir de, ou est constitué par une photo, en particulier une photo panoramique, ou un film. Dans un mode de réalisation préféré, le modèle actualisé est constitué par au moins une photo, de préférence par au moins trois photos, correspondant à une vue de face, une vue à droite et une vue à gauche des dents du patient. Des photos peuvent être prises soit pour l'arcade supérieure, soit pour l'arcade inférieure, soit pour les deux.

L'appareil personnel fournit de préférence des images en couleurs, et/ou des images infrarouges de la bouche du patient, voire du visage du patient. Les images infrarouges permettent avantageusement de faire apparaître les dents avec un excellent contraste.

Comme représenté sur la figure 2, l'appareil personnel 18 comporte un module d'acquisition 20, ou « de capture », et de préférence un module de traitement 22, de préférence encore un module de comparaison 24, et de préférence un module de communication 25.

De préférence, l'appareil personnel du patient comporte un applicatif spécialisé comportant un ou plusieurs de ces modules, de préférence l'ensemble de ces modules. De préférence encore, cet applicatif gère les rappels et informe le patient de la nécessité de créer un modèle actualisé.

De préférence, l'applicatif spécialisé est chargé dans l'appareil personnel à partir d'un support physique comme une clé USB ou un CD-ROM, ou est téléchargé sur internet ou par voie hertzienne. Dans un mode de réalisation, l'applicatif spécialisé est fourni au patient par le cabinet et/ou le laboratoire d'orthodontie. Il peut en particulier prendre la forme d'un applicatif du type de ceux couramment téléchargés sur les iPhones de la marque Apple® ou les appareils de toutes marques mettant en oeuvre les systèmes d'exploitation Android® ou tout autre système d'exploitation.

**Le module d'acquisition** 20 comporte de préférence un appareil photo ou une caméra vidéo ou infrarouge, que l'utilisateur, par exemple le patient ou un de ses proches, positionne au moyen d'un viseur ou d'un écran, avant de l'actionner.

De préférence, le module d'acquisition 20 comporte des moyens détrompeurs facilitant ce positionnement.

L'utilisateur peut être guidé par des messages écrits et/ou vocaux pour l'acquisition. Par exemple, l'appareil personnel peut annoncer « prenez une photo de face », émettre un signal pour informer l'utilisateur que la photo est acceptable ou qu'au contraire, il doit refaire une photo, annoncer « prenez une photo de droite », de préférence en affichant une flèche pour orienter l'utilisateur, etc. La fin du processus d'acquisition peut être également annoncée par l'appareil. L'appareil peut également aider au positionnement de l'appareil personnel, par exemple par des messages visuels (par exemple par affichage de flèches), et/ou sonores (comme une succession de bips dont la fréquence augmente à mesure que le positionnement de l'appareil s'améliore), et/ou écrits et/ou vocaux (« plus haut », plus bas », etc.).

Les moyens détrompeurs peuvent en particulier comporter des marques de repérage qui apparaissent sur le viseur ou l'écran. Les marques de repérage peuvent par exemple comporter une ligne destinée à être alignée avec la direction générale de la jointure entre les dents supérieures et les dents inférieures lorsque les dents sont serrées par le patient, ou une ligne verticale destinée à être alignée avec la jointure entre les deux incisives supérieures. Les marques de repérage peuvent également faire référence à d'autres parties du patient. Par exemple, elles peuvent être constituées par des marques correspondant à la position des yeux ou prendre la forme d'un contour dans lequel doit être positionné la bouche ou le visage du patient. Dans un mode de réalisation, les marques de repérage correspondent à un référentiel, par exemple sous la forme d'un écarteur ou d'un dispositif de prise de vue intra-bucal, de préférence remis au patient lors d'un rendez-vous avec son orthodontiste ou dentiste, que le patient doit positionner dans une position prédéterminée lors de l'acquisition. Par exemple, le référentiel peut être destiné à être mordu par le patient.

Dans un mode de réalisation préféré, les moyens détrompeurs sont définis, au moins partiellement, à partir d'informations fournies par le modèle cible. Par exemple, suivant les principes de la « réalité augmentée », tout ou partie du modèle cible, éventuellement retraité, peut être rendu visible sur ledit écran ou ledit viseur lors de l'acquisition.

Le retraitement du modèle cible pour faciliter l'acquisition, ou « retraitement d'acquisition », peut par exemple comprendre la création d'une ou plusieurs images ou d'une vue tridimensionnelle dans laquelle les dents apparaissent en transparence. Il peut aussi comprendre la génération d'images en deux dimensions, notamment à partir d'un modèle cible en trois dimensions, par exemple la génération d'une vue de face, d'une vue de droite et d'une vue de gauche.

Le retraitement d'acquisition peut être effectué, par exemple sur un serveur dédié, de préférence par un professionnel des soins dentaires, en particulier immédiatement après la création du modèle cible. Avantageusement, le professionnel des soins dentaires peut disposer d'une puissance de calcul permettant d'obtenir des moyens détrompeurs perfectionnés. Le retraitement d'acquisition est de préférence effectué par l'appareil personnel, en particulier avec l'applicatif spécialisé.

De préférence, l'information issue du modèle cible et utilisée pour créer les moyens détrompeurs est téléchargée dans l'appareil personnel 18.

De préférence, au moins une partie du modèle cible apparaît en transparence à l'utilisateur lors de l'acquisition. Dans un mode de réalisation, seul le contour des dents apparaît à l'utilisateur. Il est ainsi très facile pour ce dernier de superposer le modèle cible qui apparaît en transparence avec les dents du patient qu'il doit prendre en photo ou filmer. En particulier, lorsque la création du modèle actualisé nécessite de prendre une ou plusieurs photos, de préférence des photos vue de face, vue de droite et vue de gauche, l'écran ou le viseur affiche de préférence une vue correspondante du modèle cible, de préférence une représentation filaire.

Si l'acquisition est faite en vidéo ou en panoramique, un prérendu en temps réel peut être fait pendant l'acquisition. Ce prérendu guidera l'utilisateur sur les zones manquantes et/ou sélectionnera les photos ou les images vidéo les plus nettes ou mieux interpolées pour construire le modèle actualisé.

De préférence, **le module de traitement** 22 est configuré pour transformer l'information acquise par le module d'acquisition, par exemple des photos, en un modèle actualisé pouvant être comparé avec le modèle cible. Le module de traitement 22 peut par exemple traiter les images acquises afin de créer un modèle actualisé numérique, de préférence en trois dimensions.

De préférence, le module de traitement 22 comporte également des moyens pour affiner les informations acquises. Par exemple, en comparant les informations acquises et des informations issues du modèle cible, il peut détecter des déformations d'une image résultant d'un positionnement inadapté de l'objectif de l'appareil photo, positionné par exemple en contre-plongée. Dans un mode de réalisation, des informations provenant d'un ou plusieurs modèles actualisés antérieurs sont utilisées pour affiner les informations acquises.

**A l'étape c),** une comparaison est effectuée entre le modèle actualisé et le modèle cible.

Un prétraitement du modèle cible peut être nécessaire à cet effet. Par exemple, lorsque le modèle actualisé comporte de préférence une photo, le module de traitement 22 comporte des moyens pour identifier, dans le modèle cible, la vue présentant le plus de similitudes avec ladite photo. Cette vue est alors comparée à ladite photo.

La comparaison entre les modèles cible et actualisé peut être réalisée par 2 types d'algorithme, bien connus :
- Algorithmes de comparaison entre des représentations 2D, telles que des photos : Ces algorithmes comparent des photos prises selon le même point de vue et/ou déduites par analyse d'un modèle 3D, notamment pour déterminer une vue de ce modèle correspondant à l'orientation souhaitée.
- Algorithmes de comparaison entre deux modèles tridimensionnels : Un modèle tridimensionnel cible est alors comparé à un modèle actualisé tridimensionnel, par exemple obtenu par extrapolation 3D de représentations 2D. Cette comparaison peut être réalisée automatiquement ou semi-automatiquement par des algorithmes dits « de best-fit 3D » ou manuellement avec des outils d'alignement 3D, notamment par sélection, sur chacun des modèles, de trois points correspondant à des emplacements identiques. En variante, on peut calculer la différence de distance entre des points caractéristiques sur le modèle cible et la distance entre ces mêmes points caractéristiques sur le modèle actualisé. Ces points caractéristiques sont de préférence les pointes cuspidiennes des dents du patient.

De préférence, le modèle cible est chargé dans l'appareil personnel par l'organisme qui l'a créé, ce qui limite les risques de divulgation d'informations personnelles. La comparaison peut alors être effectuée par l'applicatif spécialisé.

La comparaison fournit classiquement une "distance" entre les deux modèles.

Cette distance peut être interprétée directement par le patient, l'orthodontiste ou le dentiste. De préférence, le module de comparaison 24 compare ladite distance avec un seuil d'acceptabilité et fournit une information pratique. En particulier, si la distance est inférieure au seuil d'acceptabilité, l'information pratique peut être qu'aucune action n'est à engager alors que si la distance est supérieure au seuil d'acceptabilité, l'information pratique peut être de prévoir une visite de contrôle chez le dentiste ou chez l'orthodontiste.

Dans un mode de réalisation, le module de comparaison fournit différentes informations pratiques en fonction du positionnement de ladite distance par rapport à plusieurs seuils. Par exemple, lorsque la distance dépasse un seuil critique, l'information pratique peut consister à informer de la nécessité de consulter d'urgence le dentiste ou l'orthodontiste.

Le ou les seuils utilisés par le module de comparaison peuvent être prédéterminé(s). Dans un mode de réalisation, ils sont paramétrables. De préférence, ils peuvent être fixés et modifiés à tout moment, en particulier par un professionnel des soins dentaires.

En fonction de l'information pratique reçue, le patient peut se rendre chez l'orthodontiste ou le dentiste afin de recevoir un traitement thérapeutique ou prophylactique.

De préférence, la mise en oeuvre d'une étape c) engendre immédiatement l'engagement d'un nouveau délai pour réaliser de nouvelles étapes b) et c). Avantageusement, le contrôle de la dérive éventuelle des dents est ainsi permanent.

Dans un mode de réalisation, l'information pratique est utilisée pour modifier l'intervalle de temps après lequel le patient devra être averti qu'un nouveau modèle actualisé doit être créé.

Dans un mode de réalisation, l'appareil individuel permet d'afficher des images, voire une séquence d'images montrant le positionnement des dents à différentes dates. Ces images peuvent être présentées sous la forme d'une animation.

**Le module de communication** 25 est optionnel, en particulier si, à l'étape a), le modèle cible et l'applicatif spécialisé ont été chargés dans l'appareil personnel.

Il permet à l'appareil personnel 18 de recevoir les rappels destinés au patient, mais aussi des informations utiles pour les autres modules, par exemple des informations relatives au modèle cible, notamment pour créer des marques de repérage. Le module de communication 25 permet également de transmettre, si nécessaire, le modèle actualisé et/ou les résultats de la comparaison effectuée à l'étape c), et notamment les informations pratiques, en particulier à un dentiste ou à un orthodontiste.

Ces informations peuvent être transmises immédiatement ou à des moments prédéterminés. Par exemple les modèles actualisés peuvent être envoyés à l'orthodontiste, notamment sous la forme de rapports, à une fréquence paramétrable.

Le module de communication 25 est de préférence configuré pour transmettre et/ou recevoir des données de manière sécurisée.

La communication peut être par exemple effectuée, au moins en partie, par la voie hertzienne, de préférence suivant au moins un protocole choisi parmi les protocoles edge, 3G, 4G, udmsa, hpdmsa, bluetooth, et wifi, ou par tout autre protocole, actuel ou futur, adapté aux équipements mobiles ou nomades, par synchronisation filaire avec l'ordinateur personnel, ou par transmission optique.

Comme cela apparaît clairement à présent, un procédé selon l'invention permet un contrôle précis et efficace de l'évolution du positionnement des dents du patient, sensiblement sans contrainte pour le patient. Le patient peut donc facilement mettre en oeuvre ce procédé et le risque de récidive est donc substantiellement diminué.

En aucun cas, ce procédé n'intervient dans le maintien des dents dans leur position de fin de traitement. Il ne remplace pas la pose d'une contention de fin de traitement.

Bien entendu, l'invention n'est pas limitée aux modes de réalisation décrits ci-dessus et représentés.

De préférence, le traitement des images acquises et la comparaison sont effectués par le même appareil personnel que celui qui effectue l'acquisition. De préférence encore, le modèle cible est chargé dans cet appareil personnel, de préférence par le cabinet d'orthodontie ou le laboratoire d'orthodontie qui a créé le modèle cible. Avantageusement, on limite ainsi les communications de données confidentielles.

Plusieurs appareils différents peuvent cependant être également mis en oeuvre. Par exemple, l'acquisition peut être effectuée avec un téléphone mobile et le traitement et la comparaison par un ordinateur fixe.

Par ailleurs, le traitement des images capturées et/ou la comparaison ne sont pas nécessairement mis en oeuvre dans l'appareil personnel. En particulier, un ou plusieurs de ces modules peuvent être mis en oeuvre dans un cabinet dentaire, un cabinet orthodontique ou un laboratoire orthodontique. Par exemple, dans un mode de réalisation, l'étape b) peut être mise en oeuvre par le dentiste ou l'orthodontiste.

Une acquisition par le dentiste ou l'orthodontiste permet avantageusement un bon positionnement du patient, ce qui assure des prises de vues précises et améliore la qualité de la comparaison.

En outre, avantageusement, la sécurité des transferts d'informations relatives au modèle cible ou au modèle actualisé (notamment si la comparaison est effectuée à l'extérieur des locaux du dentiste ou de l'orthodontiste) en est améliorée.

De préférence, le module d'acquisition et/ou le module de traitement et/ou le module de comparaison sont alors intégrés dans l'équipement professionnel du dentiste ou de l'orthodontiste. De préférence encore, l'acquisition est effectuée dans des positions prédéterminées, de préférence de manière automatique, c'est-à-dire sans intervention du dentiste ou de l'orthodontiste.

Pour le dentiste, la mise en oeuvre du procédé permet avantageusement de détecter des déplacements imperceptibles à l'oeil, par exemple une perte d'expansion, ou des déplacements des dents par rapport à un modèle cible qui ne correspond pas à un positionnement idéal, mais, par exemple, au meilleur positionnement que le traitement orthodontique a pu produire.

Un procédé selon l'invention peut être également utilisé à des fins d'études.

Enfin, le patient n'est pas limité à un être humain. En particulier, un procédé selon l'invention peut être utilisé pour un autre animal.

## Revendications

1. Procédé de contrôle du positionnement de dents d'un patient afin de contrôler la récidive après un traitement orthodontique, ledit procédé comportant les étapes suivantes :
a) moins de 6 mois après la fin dudit traitement orthodontique, modélisation d'un positionnement cible desdites dents sous la forme d'un modèle cible (8) au moyen d'un appareil professionnel (9), le modèle cible (8) étant un modèle des dents dans leur position corrigée, résultant de la mise en oeuvre du traitement orthodontique ;
b) après un intervalle de temps, modélisation d'un positionnement actualisé desdites dents sous la forme d'un modèle actualisé (16) au moyen d'un appareil personnel (18);
c) comparaison, par un programme d'ordinateur, desdits modèles cible et actualisé.

2. Procédé selon la revendication précédente, dans lequel le modèle cible est un modèle tridimensionnel.

3. Procédé selon l'une quelconque des revendications précédentes, dans lequel à l'étape a), la modélisation est effectuée pour que le modèle cible fournisse une information sur le positionnement des dents avec une erreur inférieure à 1/10 mm.

4. Procédé selon l'une quelconque des revendications précédentes, dans lequel l'intervalle de temps est supérieur à 1 mois.

5. Procédé selon l'une quelconque des revendications précédentes, dans lequel l'étape a) est réalisée au moyen d'un scanner 3D et l'étape b) est réalisée au moyen d'un téléphone mobile ou d'une tablette ou d'un ordinateur personnel.

6. Procédé selon l'une quelconque des revendications précédentes, dans lequel on utilise un appareil individuel choisi dans le groupe formé par un appareil photo connecté, une montre intelligente, une tablette numérique, un scanner 3D portable et un ordinateur couplé un système d'acquisition d'images, telle une webcam ou un appareil photo numérique, pour mettre en oeuvre l'étape b) et/ou l'étape c).

7. Procédé selon la revendication immédiatement précédente, dans lequel, préalablement à l'étape c), de préférence préalablement à l'étape b), le patient ou un professionnel des soins dentaires charge le modèle cible dans ledit appareil individuel.

8. Procédé selon l'une quelconque des revendications précédentes, dans lequel le modèle actualisé comporte une vidéo ou une photo des dents du patient, de préférence une photo prise face au patient, une photo prise du côté droit du patient et une photo prise du côté gauche du patient.

9. Procédé selon l'une quelconque des revendications précédentes, dans lequel, à l'étape b), une ou plusieurs photos ou une ou plusieurs vidéos des dents du patient sont traitées afin de créer un modèle actualisé tridimensionnel.

10. Procédé selon l'une quelconque des revendications précédentes, dans lequel, à l'étape c), on génère une information dont le contenu dépend de la différence entre les modèles cible et actualisé et, de préférence, on transmet cette information audit patient et/ou à un professionnel des soins dentaires.

11. Procédé selon l'une quelconque des revendications précédentes, dans lequel, en fonction de la comparaison à l'étape c), un module de communication d'un appareil personnel du patient informe le patient et/ou un professionnel des soins dentaires, de la probable nécessité d'une consultation d'un dentiste ou d'un orthodontiste.

12. Procédé selon l'une quelconque des revendications précédentes, dans lequel un téléphone mobile ou une tablette ou un ordinateur personnel du patient transmet le modèle actualisé et/ou les résultats de la comparaison effectuée à l'étape c) à un dentiste ou à un orthodontiste.

13. Procédé selon l'une quelconque des revendications précédentes, dans lequel l'appareil personnel (18) comporte des moyens d'aide au positionnement dudit appareil personnel par des messages visuels et/ou sonores et/ou écrits et/ou vocaux.

14. Procédé selon l'une quelconque des revendications précédentes, dans lequel l'appareil personnel (18) comporte un module d'acquisition (20), le module d'acquisition comportant un appareil photo ou une caméra vidéo ou infrarouge positionnable, avant actionnement dudit appareil photo ou caméra, au moyen d'un viseur ou d'un écran, le module d'acquisition comportant des moyens détrompeurs pour faciliter ledit positionnement, les moyens détrompeurs étant définis, au moins partiellement, à partir d'informations fournies par le modèle cible.

15. Procédé selon l'une quelconque des revendications précédentes, dans lequel
- l'appareil personnel (18) acquiert et traite des images à l'étape b), et effectue la comparaison à l'étape c), ou
- l'acquisition des images est effectuée avec un téléphone mobile et le traitement des images et la comparaison sont effectués par un ordinateur fixe.

16. Procédé selon l'une quelconque des revendications précédentes, dans lequel, à l'étape c), la comparaison entre les modèles cible et actualisé est réalisée par un algorithme choisi parmi :
- les algorithmes de comparaison entre des représentations 2D ;
- les algorithmes de comparaison entre deux modèles tridimensionnels.

17. Procédé selon l'une quelconque des revendications précédentes, pour
- contrôler l'évolution du positionnement des dents, et/ou déterminer une vitesse d'évolution d'un changement de position de dents, et/ou
- optimiser la date de prise de rendez-vous chez un orthodontiste ou un dentiste, et/ou
- évaluer l'efficacité d'un traitement orthodontique.

18. Appareil personnel, de préférence téléphone mobile, dans lequel est chargé
- un modèle cible, créé moins de 6 mois après la fin d'un traitement orthodontique de dents d'un patient, modélisant un positionnement cible desdites dents dans leur position corrigée résultant de la mise en oeuvre du traitement orthodontique ;
- un applicatif spécialisé apte à mettre en oeuvre les étapes b) et c) d'un procédé selon l'une quelconque des revendications 1 à 17.

19. Système comportant un appareil (9) équipant un professionnel des soins dentaires et apte à mettre en oeuvre l'étape a) d'un procédé selon l'une quelconque des revendications 1 à 17, de préférence pour créer un modèle tridimensionnel de la dentition dudit patient, un appareil personnel (18) équipant un patient, de préférence un téléphone mobile, chargé avec un applicatif spécialisé apte à mettre en oeuvre des étapes b) et c) d'un procédé selon l'une quelconque des revendications 1 à 17.

## Patentansprüche

1. Verfahren zur Überwachung der Position von Zähnen eines Patienten, um ein Rezidiv nach einer orthodontischen Behandlung zu überwachen, wobei das Verfahren die folgenden Schritte umfasst:
a) weniger als 6 Monate nach dem Ende der orthodontischen Behandlung, Modellierung einer Zielposition der Zähne in der Form eines Zielmodells (8) mit einer professionellen Vorrichtung (9), wobei das Zielmodell (8) ein Modell der Zähne in ihrer korrigierten Position ist, die aus der Durchführung der orthodontischen Behandlung resultiert;
b) nach einem Zeitintervall, Modellierung einer aktualisierten Position der Zähne in der Form eines aktualisierten Modells (16) mit einer persönlichen Vorrichtung (18);
c) Vergleich, durch ein Computerprogramm, des Ziel- und aktualisierten Modells.

2. Verfahren nach dem vorhergehenden Anspruch, wobei das Zielmodell ein dreidimensionales Modell ist.

3. Verfahren nach einem der vorhergehenden Ansprüche, wobei, in dem Schritt a), die Modellierung derart durchgeführt wird, dass das Zielmodell eine Information über die Position der Zähne mit einem Fehler von weniger als 1/10 mm liefert.

4. Verfahren nach einem der vorhergehenden Ansprüche, wobei das Zeitintervall mehr als 1 Monate beträgt.

5. Verfahren nach einem der vorhergehenden Ansprüche, wobei der Schritt a) mit einem 3D Scanner durchgeführt wird, und der Schritt b) mit einem Mobiltelefon oder einem Tablet oder einem Personalcomputer durchgeführt wird.

6. Verfahren nach einem der vorhergehenden Ansprüche, wobei eine individuelle Vorrichtung verwendet wird, ausgewählt aus der Gruppe gebildet durch einen angeschlossenen Fotoapparat, eine intelligente Armbanduhr, ein Digital-Tablet, einen tragbaren 3D Scanner und einen Computer, der mit einem System zur Erfassung von Bildern verbunden ist, wie eine Webcam oder einen Digital-Fotoapparat, um den Schritt b) und/oder den Schritt c) durchzuführen.

7. Verfahren nach einem der vorhergehenden Ansprüche, wobei, vor dem Schritt c), vorzugsweise vor dem Schritt b), der Patient oder ein zahnmedizinischer Fachmann das Zielmodell in die individuelle Vorrichtung lädt.

8. Verfahren nach einem der vorhergehenden Ansprüche, wobei das aktualisierte Modell ein Video oder ein Foto der Zähne des Patienten umfasst, vorzugsweise ein Foto, das von vorne von dem Patienten aufgenommen wird, ein Foto, das von der rechten Seite des Patienten aufgenommen wird, und ein Foto, das von der linken Seite des Patienten aufgenommen wird.

9. Verfahren nach einem der vorhergehenden Ansprüche, wobei, in dem Schritt b), ein oder mehrere Fotos oder ein oder mehrere Videos der Zähne des Patienten bearbeitet werden, um ein dreidimensionales aktualisiertes Modell zu erzeugen.

10. Verfahren nach einem der vorhergehenden Ansprüche, wobei, in dem Schritt c), eine Information generiert wird, deren Inhalt von der Differenz zwischen dem Ziel- und aktualisierten Modell abhängig ist, und vorzugsweise diese Information zu dem Patienten und/oder zu einem zahnmedizinischen Fachmann übertragen wird.

11. Verfahren nach einem der vorhergehenden Ansprüche, wobei, als Funktion des Vergleichs in dem Schritt c), ein Kommunikationsmodul einer persönlichen Vorrichtung des Patienten den Patienten und/oder einen zahnmedizinischen Fachmann über die wahrscheinliche Notwendigkeit einer Konsultation eines Zahnarztes oder eines Orthodontisten informiert.

12. Verfahren nach einem der vorhergehenden Ansprüche, wobei ein Mobiltelefon oder ein Tablet oder ein Personalcomputer des Patienten das aktualisierte Modell und/oder die Ergebnisse des Vergleichs, der in dem Schritt c) durchgeführt wird, zu einem Zahnarzt oder zu einem Orthodontisten überträgt.

13. Verfahren nach einem der vorhergehenden Ansprüche, wobei die persönliche Vorrichtung (18) Mittel zur Unterstützung der Positionierung der persönlichen Vorrichtung durch visuelle und/oder Ton- und/oder schriftliche und/oder Sprachnachrichten umfasst.

14. Verfahren nach einem der vorhergehenden Ansprüche, wobei die persönliche Vorrichtung (18) ein Erfassungsmodul (20) umfasst, wobei das Erfassungsmodul einen Fotoapparat oder eine Video- oder Infrarotkamera umfasst, die vor der Betätigung des Fotoapparats oder der Kamera mit einem Sucher oder einem Bildschirm positionierbar sind, wobei das Erfassungsmodul Ausrichtungsmittel umfasst, um die Positionierung zu erleichtern, wobei die Ausrichtungsmittel mindestens teilweise durch die Informationen definiert werden, die von dem Zielmodell geliefert werden.

15. Verfahren nach einem der vorhergehenden Ansprüche, wobei:
- die persönliche Vorrichtung (18) die Bilder aus dem Schritt b) erfasst und bearbeitet, und den Vergleich in dem Schritt c) durchführt, oder
- die Erfassung der Bilder mit einem Mobiltelefon durchgeführt wird, und die Bearbeitung der Bilder und der Vergleich mit einem stationären Computer durchgeführt werden.

16. Verfahren nach einem der vorhergehenden Ansprüche, wobei, in dem Schritt c), der Vergleich zwischen dem Ziel- und aktualisierten Modell durch einen Algorithmus durchgeführt wird, ausgewählt aus:
- Algorithmen zum Vergleichen zwischen 2D Darstellungen;
- Algorithmen zum Vergleichen zwischen zwei dreidimensionalen Modellen.

17. Verfahren nach einem der vorhergehenden Ansprüche zum:
- Überwachen der Entwicklung der Position der Zähne, und/oder Bestimmen einer Entwicklungsgeschwindigkeit einer Veränderung der Position der Zähne, und/oder
- Optimieren des Datums des Aufsuchens eines Orthodontisten oder eines Zahnarztes, und/oder
- Evaluieren der Wirksamkeit einer orthodontischen Behandlung.

18. Persönliche Vorrichtung, vorzugsweise ein Mobiltelefon, in die/das geladen werden:
- ein Zielmodell, das mindestens 6 Monate nach dem Ende einer orthodontischen Behandlung von den Zähnen eines Patienten erzeugt wird, wobei eine Zielposition der Zähne in ihrer korrigierten Position modelliert wird, die aus der Durchführung der orthodontischen Behandlung resultiert;
- eine spezialisierte Anwendung, die geeignet ist, die Schritte b) und c) eines Verfahrens nach einem der Ansprüche 1 bis 17 durchzuführen.

19. System, umfassend eine Vorrichtung (9), mit der ein zahnmedizinischer Fachmann ausgestattet ist, und die geeignet ist, den Schritt a) eines Verfahrens nach einem der Ansprüche 1 bis 17 durchzuführen, vorzugsweise um ein dreidimensionales Modell des Gebisses des Patienten zu erzeugen, und eine persönliche Vorrichtung (18), mit der ein Patient ausgestattet ist, vorzugsweise ein Mobiltelefon, auf das eine spezialisierte Anwendung geladen ist, die geeignet ist, die Schritte b) und c) eines Verfahrens nach einem der Ansprüche 1 bis 17 durchzuführen.

## Claims

1. Method for monitoring the position of a patient's teeth in order to monitor a relapse after an orthodontic treatment, said method comprising the following steps:
a) less than 6 months after the end of said orthodontic treatment, modelling a target position of said teeth in the form of a target model (8) by means of a professional apparatus (9), the target model (8) being a model of the teeth in their corrected position resulting from the orthodontic treatment;
b) after a time interval, modelling an updated position of said teeth in the form of an updated model (16) by means of a personal apparatus (18);
c) comparing said target model and updated model by means of a computer program.

2. Method according to the preceding claim, in which the target model is a three-dimensional model.

3. Method according to either of the preceding claims, in which, at step a), the modelling is carried out such that the target model supplies information concerning the position of the teeth with an error of less than 1/10 mm.

4. Method according to any one of the preceding claims, in which the time interval is longer than 1 month.

5. Method according to any one of the preceding claims, in which step a) is carried out by means of a 3D scanner, and step b) is carried out by means of a cell phone or a tablet or a personal computer.

6. Method according to any one of the preceding claims, in which use is made of an individual apparatus chosen from the group comprising a connected photographic apparatus, a smart watch, a digital tablet, a portable 3D scanner and a computer linked to an image acquisition system, such as a webcam or a digital photographic apparatus, in order to implement step b) and/or step c).

7. Method according to the immediately preceding claim, in which, prior to step c), preferably prior to step b), the patient or a dental professional loads the target model onto said individual apparatus.

8. Method according to any one of the preceding claims, in which the updated model comprises a video or a photo of the patient's teeth, preferably a photo of the patient from the front, a photo taken from the patient's right and a photo taken from the patient's left.

9. Method according to any one of the preceding claims, in which, at step b), one or more photos or one or more videos of the patient's teeth are processed in order to create a three-dimensional updated model.

10. Method according to any one of the preceding claims, in which information is generated at step c), the content of which information depends on the difference between the target model and updated model, and this information is preferably transmitted to said patient and/or to a dental professional.

11. Method according to any one of the preceding claims, in which, depending on the comparison at step c), a communication module of a personal apparatus of the patient's informs the patient and/or a dental professional of the probable need for a consultation with a dentist or an orthodontist.

12. Method according to any one of the preceding claims, in which a cell phone or a tablet or a personal computer of the patient's transmits the updated model and/or the results of the comparison carried out at step c) to a dentist or to an orthodontist.

13. Method according to any one of the preceding claims, in which the personal apparatus (18) has means to help in the positioning of said personal apparatus via visual messages and/or audio messages and/or written messages and/or voice messages.

14. Method according to any one of the preceding claims, in which the personal apparatus (18) has an acquisition module (20), the acquisition module having a photographic apparatus or a video or infrared camera that can be positioned, before actuation of said photographic apparatus or camera, by means of a viewfinder or a screen, the acquisition module having error prevention means to facilitate said positioning, the error prevention means being defined, at least partially, on the basis of information supplied by the target model.

15. Method according to any one of the preceding claims, in which
- the personal apparatus (18) acquires and processes images at step b) and performs the comparison at step c), or
- the acquisition of the images is performed with a cell phone, and the processing of the images and the comparison are performed by a fixed computer.

16. Method according to any one of the preceding claims, in which, at step c), the comparison between the target model and updated model is performed by an algorithm chosen from among:
- algorithms for comparison between 2D representations;
- algorithms for comparison between two three-dimensional models.

17. Method according to any one of the preceding claims to
- control a change in the position of the teeth, and/or determine a speed of change in the position of teeth, and/or
- optimize the scheduling of visits to an orthodontist or a dentist, and/or
- evaluate the efficacy of an orthodontic treatment.

18. Personal apparatus, preferably a cell phone, in which is loaded
- a target model, created less than 6 months after the end of orthodontic treatment of teeth of a patient, modelling a target position of said teeth in their corrected position resulting from the orthodontic treatment;
- a specialized app that is able to implement steps b) and c) of a method according to any one of Claims 1 to 17.

19. System comprising an apparatus (9) with which a dental professional is equipped and which is able to implement step a) of a method according to any one of Claims 1 to 17, preferably in order to create a three-dimensional model of the dentition of said patient,
a personal apparatus (18) with which a patient is equipped, preferably a cell phone, and on which a specialized app is loaded that is able to implement steps b) and c) of a method according to any one of Claims 1 to 17.
